# EUROPEAN PATENT APPLICATION

(11) **EP 1 661 984 A1**
(43) Date of publication of application: **31.05.2006**
(21) Application number: 04772412.5
(22) Date of filing: 24.08.2004
(51) Int. Cl.: C12N 5/00, A01K 67/027, A61K 35/14, A61P 7/00, A61P 35/02, C12N 15/00

(54) **METHOD OF DIFFERENTIATION FROM EMBRYO-STEM CELL OF PRIMATE TO HEMATOGENOUS CELL**

(30) Priority: 25.08.2003 JP 2003208724
(71) Applicant: TANABE SEIYAKU CO., LTD., Chuo-ku, Osaka-shi, Osaka 541-8505 (JP)
(72) Inventor: HANAZONO, Yutaka, Kawachi-gun, Tochigi 329-0433 (JP); SASAKI, Kyoko, Itabashi-ku, Tokyo 173-0003 (JP)
(74) Representative: HOFFMANN EITLE
(86) International application number: PCT/JP2004/012456
(87) International publication number: WO 2005/019441

(57) **Abstract**

A method of differentiation from an embryonic stem cell of a primate into a hematopoietic cell, comprising maintaining an embryonic stem cell of a primate under conditions suitable for induction of differentiation into a hematopoietic cell, transplanting the resulting cell into a fetus in a uterus of a pregnant sheep, rearing the fetus, administering a cytokine specific for a primate to a born lamb, and obtaining a hematopoietic cell of a primate from a sheep obtained by rearing the lamb, a method for producing a hematopoietic cell of a primate, a hematopoietic cell obtained by the method, and a method for producing a chimeric sheep which produces a hematopoietic cell of a primate, comprising maintaining an embryonic stem cell of a primate under conditions suitable for induction of differentiation into a hematopoietic cell, and transplanting the resulting cell into a fetus in a uterus of a pregnant sheep. According to the present invention, it becomes possible to develop a means for treating a disease or a condition requiring construction of a hematopoietic system or an action of a hematopoietic cell.

SEQUENCE LISTING

<110> TANABE SEIYAKU CO., LTD.

<120> A method for differentiating a primate embryonic stem cell into a hemopoietic cell

<130> 04-054-PCTJP

<150> JP 2003-208724
<151> 2003-08-25

<160> 5

<170> PatentIn version 3.2

<210> 1
<211> 20
<212> DNA
<213> Artificial

<220>
<223> a sequence of primer CB2MG-2F

<400> 1
gtctggattt catccatctg                                        20

<210> 2
<211> 21
<212> DNA

<213> Artificial

<220>
<223> a sequence of primer hB2MG-5R

<400> 2
ggctgtgaca aagtcacatg g                                    21

<210> 3
<211> 22
<212> DNA
<213> Artificial

<220>
<223> a sequence of a primer hB2MG-3R

<400> 3
ggtgaattca gtgtagtaca ag                                   22

<210> 4
<211> 24
<212> DNA
<213> Artificial

<220>
<223> a sequence of primer C beta1

<400> 4

cattgtcatg gactctggcg acgg                                    24

<210>  5

<211>  24

<212>  DNA

<213>  Artificial

<220>

<223>  a sequence of primer C beta2

<400>  5

catctcctgc tcgaagtcta gggc                                    24

## Description

### TECHNICAL FIELD

The present invention relates to a technique for supplying a hematopoietic cell, a differentiated cell and the like of a primate. More particularly, the present invention relates to a method of differentiation from an embryonic stem cell of a primate into a hematopoietic cell, a hematopoietic cell obtained by the differentiation method, and a method for producing a chimeric sheep which produces a hematopoietic cell of a primate.

### BACKGROUND ART

To a patient with a disease such as aplastic leukemia, hematopoietic stem cell transplantation represented by bone marrow transplantation, peripheral blood stem cell transplantation, cord blood stem cell transplantation and the like is performed, to produce a hemocyte in a body of a patient.

However, in the aforementioned hematopoietic stem cell transplantation, there is a disadvantage that it is difficult to stably supply a hematopoietic stem cell compatible with a patient to be subjected to the transplantation, that a donor may undergo physical or bodily damage upon collection of a hematopoietic stem cell, or the like.

Then, in order to stably supply a hematopoietic stem cell, an attempt has been made to transplant a human hematopoietic stem cell into a pig fetus via a uterus, and to amplify a hematopoietic precursor cell in a pig body [Hiromitsu Nakauchi and one other, "3. Establishment of Human Blood Chimeric Pig And Its Application", The Japanese Association of Medical Sciences 117^{th} Symposium Record, Stem Cell And Cell Therapy-[III] Tissue and Organ Stem Cell: Fundamental Development for Clinical Applications (2) held on August 4 - 6, 2000, p99-106 [online], internet <URL:
http://www.med.or.jp/jams/symposium/kiroku/117/pdf/117099.pdf>].

On the other hand, in order to apply to regenerative medicine for the purpose of functional recovery, repair, regeneration and the like of a tissue or an organ having dysfunction, differentiation of an embryonic stem cell (hereinafter, also referred to as ES cell) has been *studied in vitro.*

Examples of an attempt *of in vitro* differentiation from an embryonic stem cell into a hemocyte include differentiation from a mouse ES cell in which HoxB4, a homeotic selector gene involved in self regeneration of a hematopoietic stem cell is introduced, into a cell exhibiting a phenotype of a final hematopoietic stem cell (Michael Kyba et al., *Cell,* vol.109, published on April 5, 2002, p29-37), differentiation from a mouse ES cell, into a cell presumed to be a hematopoietic precursor cell utilizing an OP9 stromal cell (Toru Nakano, "5. Differentiation from Embryonic Stem cell into Hemocyte", Takashi Yokota et al Eds., *Experimental Medicine Suppl.* "Frontier of Stem Cell Study and Application to Regenerative Medicine, Elucidation of Mechanism of Development and Differentiation, and to Clinical", published on September 25, 2001, vol.19, No.15, p1966-1971; Toru Nakano et al., *Science,* vol.265, August 19, 1994, p1098-1101), differentiation of rhesus monkey ES cell into a hematopoietic precursor cell by culturing on a mouse S17 stromal cell [Fei Li et al., *Blood,* vol.98, published on July 15, 2001, p335-342], and differentiation from mouse ES cell into a hematopoietic precursor cell [Britt M. Johansson et al., *Molecular and Cellular Biology,* vo1.15, No.1, published in January, 1995, p141-151].

In addition, the aforementioned literature of Fei Li et al. discloses that, by culturing a rhesus monkey ES cell in the presence of bone morphogenetic protein 4 (BMP-4), differentiation of a hematopoietic precursor cell is increased 15-fold as compared with the case in the absence of BMP-4. Further, the aforementioned literature of Britt M. Johansson et al. discloses that, by culturing a mouse ES cell in the presence of BMP-4, differentiation of a hematopoietic precursor cell is increased as compared with the case in the absence of BMP-4.

However, generally, in contrast to cells appearing at an initial stage of development (neuron, myocardial cell; fetal hematopoietic cell etc.), there is a disadvantage that it is difficult to differentiate a cell which appears "field-" inductively at a later stage of development, such as a hematopoietic stem cell, a hepatocyte and a pancreatic β cell, from the ES cell as compared with *in vitro* differentiation.

In addition, in the differentiation method described in the aforementioned literature of Michael Kyba et al., since an ES cell into which a HoxB4 gene is introduced is used, there is a disadvantage that an application range of the resulting hematopoietic stem cell is limited in some cases.

Further, the differentiation methods described in the aforementioned literature authored by Toru Nakano [5. Differentiation from Embryonic Stem cell into Hemocyte] and the aforementioned literature of Toru Nakano *[Science]* are characterized by use of an OP9 stromal cell, but actually, it is thought that only yolk sac hematopoiesis (primary hematopoiesis) is reproduced, and there is a disadvantage that it is difficult to induce differentiation of a hematopoietic stem cell which can reconstitute a hematopoietic system.

### DISCLOSURE OF INVENTION

In one aspect, the present invention relates to a method of differentiation from an embryonic stem cell of a primate into a hematopoietic cell, which enables at least one of stable supply of a hematopoietic cell of a primate, differentiation from the embryonic stem cell of a primate into a hematopoietic cell without performing a genetic engineering procedure such as introduction of a foreign gene into an embryonic stem cell of a primate, and the like. Also, in another aspect, the present invention relates to a method for producing a hematopoietic cell of a primate, which enables at least one of stable supply of a hematopoietic cell of a primate, large scale supply of a hematopoietic cell of a primate, stable supply of platelet for transfusion of a primate, and stable supply of a CD34⁺ cell for transplantation of a primate, and the like. Further, in still another aspect, the present invention relates to a hematopoietic cell obtained by the aforementioned production method. In addition, in a still further another aspect, the present invention relates to a method for producing a chimeric sheep, which can stably supply a hematopoietic cell of a primate.

Specifically, the gist of the present invention relates to:
[1] a method of differentiation from an embryonic stem cell of a primate into a hematopoietic cell, characterized by maintaining an embryonic stem cell of a primate under conditions suitable for induction of differentiation into a hematopoietic cell, transplanting the resulting cell into a fetus in a uterus of a pregnant sheep, rearing the fetus, administering a cytokine specific for a primate to a born lamb, and obtaining a hematopoietic cell of a primate from a sheep obtained by rearing the lamb,
[2] the method according to the above [1], wherein the method comprises the steps of:
   (I) maintaining an embryonic stem cell of a primate on a feeder cell, the feeder cell being a stromal cell strain deficient in macrophage colony-stimulating factor, and
   (II) transplanting a primate-derived cell obtained in the step (I) into a fetus in a uterus of a pregnant sheep, and rearing the fetus to birth,
[3] the method according to the above [2], wherein in the step (I), an embryonic stem cell of a primate is maintained on a feeder cell in the presence of bone morphogenetic protein 4,
[4] a method for producing a hematopoietic cell of a primate, comprising the steps of:
   (I) maintaining an embryonic stem cell of a primate on a feeder cell, the feeder cell being a stromal cell strain deficient in macrophage colony-stimulating factor,
   (II) transplanting a primate-derived cell obtained in the step (I) into a fetus in a uterus of a pregnant sheep, and rearing the fetus to birth, and
   (III) administering a cytokine specific for a primate to a lamb born in the step (II), and separating a hematopoietic cell of a primate differentiated from the embryonic stem cell of a primate from a sheep obtained by rearing the lamb,
[5] a hematopoietic cell obtained by the method as defined in the above [4], and
[6] a method for producing a chimeric sheep which produces a hematopoietic cell of a primate, characterized by maintaining an embryonic stem cell of a primate under conditions suitable for induction of differentiation into a hematopoietic cell, transplanting the resulting cell into a fetus in a uterus of a pregnant sheep, administering a cytokine specific for a primate to a born lamb, and rearing the lamb.

### BRIEF DESCRIPTION OF THE DRAWINGS

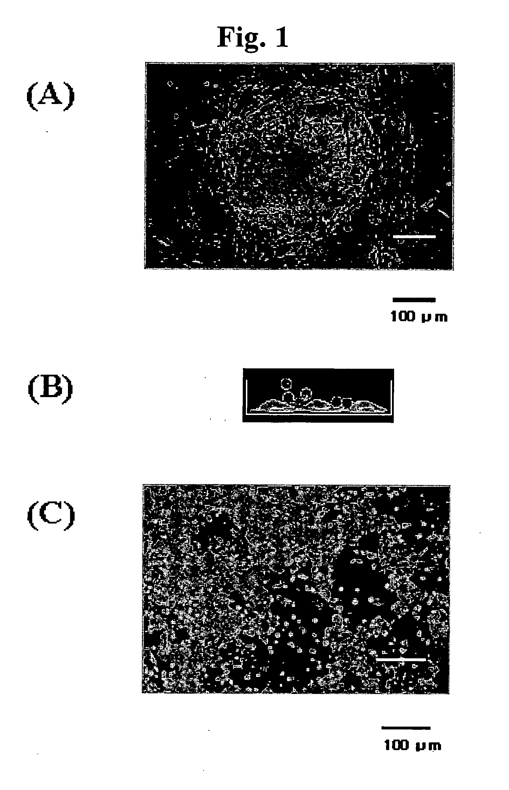

Fig. 1 shows a cell obtained by maintaining an embryonic stem cell of a primate under the conditions suitable for induction of differentiation into a hematopoietic cell. Panel (A) shows an undifferentiated embryonic stem cell, and the scale bar indicates 100 µm. Panel (B) shows a schema of a cell in the case of culturing on an OP9 cell in the presence of BMP-4. Panel (C) shows a cell obtained by maintaining an embryonic stem cell under the conditions for inducing differentiation of a hematopoietic system, and the scale bar indicates 100 µm.

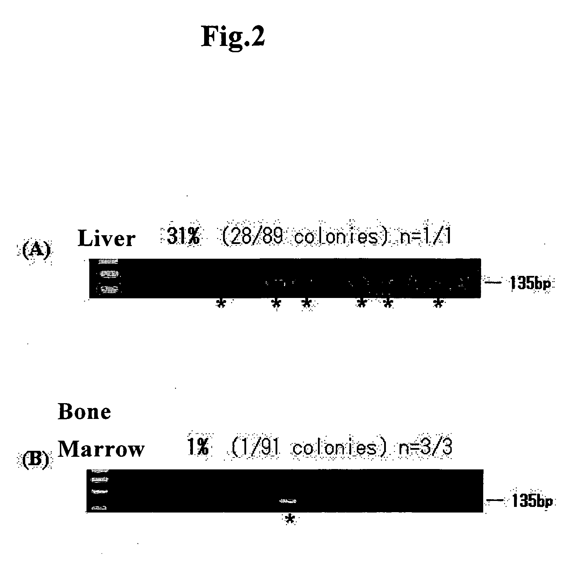

Fig. 2 shows results of detection of monkey β2-microglobulin in a sheep fetus obtained after transplantation. In the figure, Panel (A) shows results of fetal liver, and Panel (B) shows results of bone marrow.

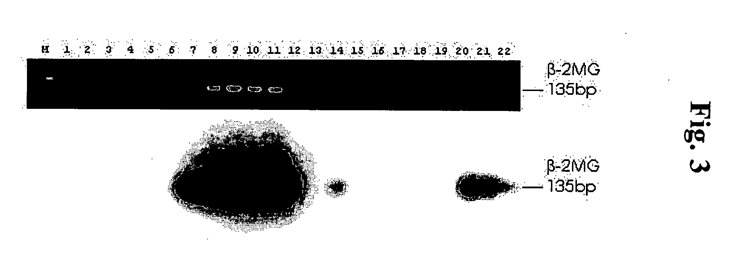

Fig. 3 shows results of detection of monkey β2-microglobulin in peripheral blood or bone marrow before and after administration of human SCF to a sheep. The upper panel shows results of PCR, and the lower panel shows results of Southern blot hybridization. In the figure, M indicates a molecular weight marker, lanes 1 to 3 indicate a negative control (water), lane 4 indicates a sheep DNA alone, lanes 5 to 10 indicate 0.0001% (the number of cells), 0.001% (the number of cells), 0.01% (the number of cells), 0.1% (the number of cells), 1% (the number of cells) and 10% (the number of cells) monkey DNAs, respectively, lane 11 indicates a monkey DNA alone, lane 12 indicates the absence of a sample, lanes 13 to 17 indicate peripheral blood-derived samples before administration of human SCF, on administration day 6, on day 1 after administration, on day 3 after administration and on day 40 after administration, respectively, and lanes 18 to 22 indicate bone marrow-derived samples before administration of human SCF, on administration day 6, on day 1 after administration, on day 3 after administration and on day 40 after administration. It shows that, by administering human SCF which acts on a monkey but not on a sheep, a monkey cell appeared in peripheral blood and bone marrow blood of the sheep.

### BEST MODE FOR CARRYING OUT THE INVENTION

The present invention is based on surprising findings of the present inventors that, by treating an embryonic stem cell under conditions for inducing differentiation into a hematopoietic cell, and transplanting the resulting cell into a sheep fetus in a uterus of a pregnant sheep, a sheep after birth produces a hematopoietic cell essentially derived from a monkey.

In one aspect, the present invention relates to a method of differentiation from an embryonic stem cell of a primate into a hematopoietic cell.

Hereinafter, in the present specification, the case wherein a sheep is used will be explained, but in the present invention, the invention can be carried out similarly in other non-human mammals (e.g. pig, horse, monkey etc). Also by using other non-human mammals, a chimeric non-human mammal can be produced in at least 1% chimera ratio, and a hematopoietic cell can be produced.

A method of differentiation from an embryonic stem cell of a primate into a hematopoietic cell of the present invention is a method characterized by maintaining an embryonic stem cell of a primate under the conditions suitable for induction of differentiation into a hematopoietic cell, transplanting the resulting cell into a fetus in a uterus of a pregnant sheep, and obtaining a hematopoietic cell of a primate from a born sheep.

A method of differentiation from an embryonic stem cell of a primate into a hematopoietic cell of the present invention is, more preferably, a method characterized by maintaining an embryonic stem cell of a primate under the conditions suitable for induction of differentiation into a hematopoietic cell, transplanting the resulting cell into a fetus in a uterus of a pregnant sheep, rearing the fetus, administering a cytokine specific for a primate to a born lamb, and obtaining a hematopoietic cell of a primate from a sheep obtained by rearing the lamb.

A great feature of the method of differentiation of the present invention is that the method comprises maintaining an embryonic stem cell of a primate under the conditions suitable for induction of differentiation into a hematopoietic cell (hereinafter, also referred to as the conditions for inducing differentiation of a hematopoietic system).

In the method of differentiation of the present invention, since an embryonic stem cell of a primate is maintained under the aforementioned conditions for inducing differentiation of a hematopoietic system, according to the method of differentiation of the present invention, surprisingly, there is exhibited an excellent effect that the embryonic stem cell of a primate can be differentiated into a hematopoietic cell although when an undifferentiated embryonic stem cell is transplanted as it is, differentiation from the undifferentiated embryonic stem cell into a hematopoietic cell is not substantially generated.

In addition, in the method of differentiation of the present invention, since an embryonic stem cell of a primate is maintained under the aforementioned conditions for inducing differentiation of a hematopoietic system, there is exhibited an excellent effect that, when the embryonic stem cell of a primate is transplanted into a sheep fetus, the internal environment of the sheep fetus can be utilized more effectively [in the strict sense, after birth, a born lamb (hereinafter, in the present specification, also expressed as "postnatal lamb")], to be differentiated.

Further, since an embryonic stem cell of a primate is differentiated into a hematopoietic cell, the method of the present invention is advantageous in that a genetic engineering procedure such as introduction of a foreign gene (e.g. homeotic selector gene) which induces differentiation of the embryonic stem cell of a primate is substantially not required. Therefore, the method of differentiation of the present invention does not require higher techniques, expensive machines, reagents or the like, and is useful in supplying a cell and a tissue for transplantation, and the like.

In addition, a great feature of the method of differentiation of the present invention resides also in use of a sheep fetus in a uterus of a pregnant sheep.

Since the sheep fetus is used, the method of differentiation of the present invention exhibits an excellent effect that a cell obtained by maintaining an embryonic stem cell of a primate under the aforementioned conditions for inducing differentiation of a hematopoietic system can be, surprisingly, differentiated into a hematopoietic cell essentially derived from a primate.

In addition, according to the method of differentiation of the present invention, since the sheep fetus is used, there can be obtained an excellent effect that a chimera ratio with a primate is increased. Therefore, according to the method of differentiation of the present invention, there is exhibited an excellent effect that a hematopoietic cell essentially derived from a primate can be more efficiently obtained in a sheep body.

Further, since a sheep fetus in a uterus of a pregnant sheep is used, the method of differentiation of the present invention has an excellent feature that immunological tolerance to a transplanted cell is obtained, and the same cell can be additionally transplanted after birth. Therefore, according to the method of differentiation of the present invention, a hematopoietic cell essentially derived from a primate can be more efficiently and stably supplied.

A great feature of the method of differentiation of the present invention resides also in that a cell obtained by maintaining an embryonic stem cell of a primate under the aforementioned conditions for inducing differentiation of a hematopoietic system is transplanted into a sheep fetus in a uterus of a pregnant sheep.

Since a cell obtained by maintaining an embryonic stem cell of a primate under the aforementioned conditions for inducing differentiation of a hematopoietic system is transplanted into a sheep fetus in a uterus of a pregnant sheep, the method of differentiation of the present invention can efficiently and stably supply a hematopoietic cell derived from a primate. Further, since a cell obtained by maintaining an embryonic stem cell of a primate under the aforementioned conditions for inducing differentiation of a hematopoietic system is transplanted into a sheep fetus in a uterus of a pregnant sheep, the method of differentiation of the present invention exhibits an excellent effect that the transplanted embryonic stem cell of a primate or a hematopoietic cell essentially derived from a primate can be selectively stimulated by a cytokine of a primate which does not act on a sheep. Therefore, according to the present invention, a hematopoietic cell derived from a primate can be more efficiently and stably supplied. Further, in the method of differentiation of the present invention, there can be exhibited an excellent effect that, by using a cell which has been maintained under the conditions for inducing differentiation of a hematopoietic system for 6 days to 8 days, preferably for 6 days, a chimeric animal can be surprisingly obtained at a high efficiency, and a surprisingly high chimera ratio can be accomplished.

In the present specification, the "primate" refers to human, monkey and the like. The monkey includes cynomolgus monkey, rhesus monkey, Japanese macaque, marmoset and the like.

In the present specification, the "embryonic stem cell (hereinafter, also referred to as ES cell)" refers to an undifferentiated cell having pluripotency and self-replicating ability.

Specific examples of an embryonic stem cell of a primate used in the method of differentiation of the present invention include a CMK6 cell and the like.

Such embryonic stem cell can be maintained and supplied, for example, by culturing in a medium for an ES cell using a cell such as mouse embryonic fibroblast which has stopped mitotic proliferation as a feeder cell.

The medium for an ES cell may be a medium suitable for the embryonic stem cell depending on the kind of an embryonic stem cell. Specifically, for example, in the case of an embryonic stem cell strain CMK6 derived from a cynomolgus monkey, examples include a medium containing 163 ml of DMEM/F12, 30 ml of fetal bovine serum (final concentration: 15%), 2 ml of L-glutamine (final concentration: 2 mM), 2 ml of penicillin (100 U/ml)-streptomycin (100 µg/ml), 2 ml of nonessential amino acid solution, and 1 ml of 2-mercaptoethanol (final concentration: 0.1 mM) as a composition per 200 ml.

Maintenance and proliferation of the embryonic stem cell in undifferentiated state vary depending on the kind of an embryonic stem cell, and can be performed, for example, by culturing under conditions of the presence of leukemia inhibitory factor (LIF), and the like.

In addition, the culturing conditions for maintenance and proliferation of the embryonic stem cell in undifferentiated state vary depending on the kind of an embryonic stem cell, and exemplified by maintenance in a gas phase of 5% by volume CO₂ at 36° to 38°C, preferably at 37°C, and the like.

The feeder cell used for maintenance and proliferation of the embryonic stem cell in undifferentiated state varies depending on the kind of cell. For example, the maintenance, proliferation and the like can be performed in a D10 medium (DMEM medium containing 10% by volume fetal bovine serum) and the like.

The culturing conditions for the feeder cell vary depending on the kind of cell, and exemplified by, for example, maintenance in a gas phase of 5% by volume CO₂ at 36° to 38°C, preferably at 37°C, and the like.

In the present specification, a hematopoietic cell includes a hematopoietic stem cell and a group of cells produced by differentiation from the hematopoietic stem cell, i.e., cells having properties of erythroblast, myelocyte, megakaryocyte, lymphocyte and the like, specifically, erythroblast, myelocyte and the like.

In the present invention, the conditions suitable for induction of differentiation into a hematopoietic cell, i.e., the conditions for inducing differentiation of a hematopoietic system include various conditions such as, for example, condition of culturing in the presence of type IV collagen, condition of culturing in the presence of an α-minimum essential medium (α-MEM) or the like, condition of culturing on a feeder cell suitable for differentiation of a hematopoietic system, and condition of formation of an embryoid body followed by adhering of the embryoid body to a dish, and condition of any combination thereof.

In the present invention, the "cell obtained by maintaining an embryonic stem cell of a primate under the conditions suitable for induction of differentiation into a hematopoietic cell" is a cell at a differentiation stage which becomes negative or weakly positive in both cases where hematopoietic colony forming ability and expression of a surface marker of a hematopoietic system are assessed. Such cell may be a so-called mesodermal cell or a cell having state, properties and the like closely similar to those of the mesodermal cell. Specifically, the cell includes a cell obtained by maintaining a cynomolgus monkey-derived embryonic stem cell strain CMK6 under the conditions suitable for induction of differentiation into a hematopoietic cell for about 6 days, and the like.

The hematopoietic colony forming ability can be assessed, for example, by colony assay described later, or the like.

The surface marker includes CD45, TER119, α₄-integrin, VE-cadherin, Sca-1, CD34, CD13, CD14, GPIIb/IIIa, CD3, CD4, CD8, sIgM, CD19, c-Kit (CD117), Thy-1 (CD90), CD31, HLA-ABC, β2-microglobulin and the like.

The feeder cell used upon maintenance under the conditions for inducing differentiation of a hematopoietic system includes a stromal cell strain deficient in macrophage-stimulating factor, for example, cells obtained by subjecting a mouse bone marrow cell strain deficient in macrophage-stimulating factor, mouse yolk sac cell strain deficient in macrophage-stimulating factor or the like to mitomycin C treatment or X-ray treatment, and the like. The stromal cell strain deficient in macrophage-stimulating factor includes an OP9 cell strain, the mouse bone marrow cell strain includes an S17 cell strain [Collins et al., J. *Immunol.,* published in 1987, vol. 138, p1082-1087], and the mouse yolk sac cell strain includes a C166 cell strain [Wang et al., *In Vitro Cell. Dev. Biol. Anim.,* published in 1996, vol. 32, p292-299], and the like.

The feeder cell used upon maintenance under the conditions for inducing differentiation of a hematopoietic system can be produced by, for example, plating a stromal cell strain deficient in macrophage-stimulating factor, for example, on a gelatin-coated culturing container containing, for example, an α-MEM medium (Minimum Essential medium Eagle), or the like. The feeder cell may be plated to such an extent that it covers the culturing container without any gap.

The culturing conditions of the feeder cell vary depending on the kind of a cell used, and in the case of a stromal cell strain OP9 cell, the conditions can be exemplified by maintenance in a gas phase of 5% CO₂ at preferably 36° to 38°C, particularly preferably at 37°C, and the like.

The feeder cell used upon maintenance under the conditions for inducing differentiation of a hematopoietic system, specifically when an OP9 cell is used as a feeder cell, can be obtained by:
- culturing on a medium for an OP9 cell {e.g. composition per 1250 ml: 980 ml of α-MEM [manufactured by Gibco, Cat. No.: 12000-022], 250 ml of fetal bovine serum (final concentration: 20%), 10 ml of penicillin (100 U/ml)-streptomycin (100 µg/ml), and 10 ml of 200 mM L-glutamine solution} under the conditions of 37°C and 5%CO₂,
- washing a cell which has grown to confluent or immediately therebefore adhering in a culture medium, with a phosphate buffered saline twice,
- treating the washed cell on a dish with trypsin,
- adding the medium for an OP9 cell, and recovering a medium containing the cell,
- subculturing the resulting cell in the medium for an OP cell at 1:4 to 1:5,
- culturing the cells to confluent under the conditions of 37°C and 5% CO₂,
- inactivating the resulting cell with mitomycin C, and
- plating the resulting cell (e.g. 1×10⁵ cells/ml) on the medium for an OP9 cell on a gelatin-coated culturing dish, followed by culturing.

It is desirable that the pregnant sheep used in the present invention is 40 days or more, preferably 50 days or more, more preferably 60 days or more pregnant, from the viewpoint of security, for example, prevention of abortion, and 85 days or less, preferably 75 days or less, more preferably 60 days or less pregnant from the viewpoints of decreasing immunological rejection and obtaining sufficient implantation ratio of a transplanted cell. Therefore, it is desirable that a fetus in a uterus is in the 50th to 70th day of pregnancy.

More specifically, the method of differentiation of the present invention includes a method comprising the steps of:
(I) maintaining an embryonic stem cell of a primate on a feeder cell, the feeder cell being a stromal cell strain deficient in macrophage colony-stimulating factor, and
(II) transplanting the cell derived from a primate obtained in the step (I) into a fetus in a uterus of a pregnant sheep, and rearing the fetus to birth.

It is desirable that, in the step (I), an embryonic stem cell of a primate is plated so as to have a high density from the viewpoint of better differentiation.

It is desirable that, in the step (I), culturing is performed on a feeder cell, in a medium in accordance with an embryonic stem cell of a primate.

Here, during the culture, the medium may be appropriately exchanged.

In addition, it is desirable that, in the step (I), an embryonic stem cell of a primate is maintained in a gas phase of 5% CO₂ at preferably 36° to 38°C, particularly preferably at 37°C on a feeder cell.

Specifically, in the case of a cynomolgus monkey embryonic stem cell strain CMK6, the conditions include those of suspending in 5 ml of a medium for differentiation [composition per 100 ml: 84 ml of IMDM (Iscove's Modified Dulbecco's Medium), 8 ml of 8% horse serum, 8 ml of 8% fetal bovine serum, 0.18 µg of 5×10⁻⁶ M hydrocortisone, 2 µg of BMP-4 (final concentration: 20 ng/ml), 2 µg of SCF (final concentration: 20 ng/ml), 2 µg of IL-3 (final concentration: 20 ng/ml), 1 µg of IL-6 (final concentration: 10 ng/ml), 2 µg of VEGF (final concentration: 20 ng/ml), 2 µg of G-CSF (final concentration: 20 ng/ml), 2 µg of Flt-3 ligand (final concentration: 10 ng/ml) and 200 U of EPO (2 U/ml)], plating an appropriate amount of cells from the resulting cell suspension on a feeder cell relative to 5×10⁵ cells and, thereafter, culturing the cell for 6 days under the conditions of 37°C and 5% CO₂.

Here, the cell obtained by performing the step (I) corresponds to the "cell obtained by maintaining an embryonic stem cell of a primate under the conditions suitable for induction of differentiation into a hematopoietic cell".

From the viewpoints of obtaining a chimeric sheep at a higher efficiency, obtaining a higher chimera ratio, and the like, it is desirable that, in the step (I), an embryonic stem cell, for example, a cynomolgus monkey embryonic stem cell strain CMK6 or the like is maintained under the conditions suitable for induction of differentiation into a hematopoietic cell for 6 days to 8 days, preferably for 6 days. Thus, it becomes possible to perform differentiation from an embryonic stem cell into a hematopoietic cell at a surprisingly high efficiency and more rapidity.

It is desirable that, from the viewpoint of obtaining a hematopoietic cell more efficiently, in the step (I), preferably, an embryonic stem cell of a primate is maintained in the presence of bone morphogenetic protein 4, and in the absence of leukemia inhibitory factor on a feeder cell, in addition to the aforementioned conditions for inducing differentiation of a hematopoietic system.

The cell obtained by performing the step (I) is then transplanted into a fetus in a uterus of a pregnant sheep, and is reared to birth [step (II)].

A cell used in transplantation can be prepared by treating a cell with trypsin, and suspending the cell in, for example, 0.1% BSA (bovine serum albumin)/HBSS (Hanks buffered saline solution) so as to have a density of, for example, 1×10⁶ cells or more, preferably 1×10⁶ cells to 1×10⁹ cells, preferably 1×10⁷ cells to 1×10⁹ cells, from the viewpoint of improvement in a implantation ratio and a chimera ratio.

In the step (II), transplantation of a cell into a fetus can be performed, for example, by injecting a cell for the transplantation intrahepatically (or in more strict sense, intrahepatoparenchymally), intraperitoneally, intracardially, intraumbilically or the like by puncture.

Transplantation can be performed, for example, by:
- acclimatizing a pregnant sheep being about 60 days pregnant to the rearing environment at transplantation one week to 10 days before transplantation in advance;
- anesthetizing the maternal sheep;
- fixing the sheep in a posture suitable for operation;
- turning an uterus out of abdominal cavity of the maternal sheep after various operations, for example, after celiotomy by middle incision of hypogastrium in the case where a cell is transplanted intraperitoneally or intrahepatoparenchymally, in order to facilitate transplantation of a cell for the transplantation into a transplantation place by a clean procedure; and the like.

As a method of transplanting a cell into a fetus in a uterus, for example, myometrium of a sheep is incised, a sheep fetus is driven into a velamen which is exposed with preserved, a cell for the transplantation is injected into a fetus transplantation site under opening through a transparent velamen by puncture, and myometrium, peritoneal muscle layer and a skin may be sequentially sutured to close an abdomen. Alternatively, a cell for the transplantation is injected into a transplantation site of a sheep fetus by puncture from above a uterus wall under ultrasonic guide without incising a uterus, and a peritoneal muscle layer and a skin may be sequentially sutured to close an abdomen.

In the step (II), after performing the transplantation, the pregnant sheep is bred until birth, for example, under the same conditions as those of normal breeding.

Production of a hematopoietic cell essentially derived from a primate by the sheep fetus obtained in the step (II), i.e., in the strict sense, a postnatal lamb, can be assessed by collecting an appropriate tissue, for example, a tissue of liver, bone marrow or the like from the sheep fetus (i.e. in the strict sense, after birth, a postnatal lamb), to obtain a cell for primary culturing and, for the resulting cell, (1) performing hematopoietic colony assay, (2) investigating a hematopoietic colony formed in the (1) on expression of a marker gene specific for a primate.

The hematopoietic colony assay of the (1) is performed, for example, by:
i) obtaining a tissue such as tissue biopsy of liver, or bone marrow from a sheep fetus (i.e. in the strict sense, after birth, a postnatal lamb),
ii) treating the resulting tissue by a proper treatment such as trypsin treatment, DNaseI treatment, and lysis treatment depending on the tissue, to obtain a cell,
iii) suspending the resulting cell in 2% by weight FBS (fetal bovine serum)-IMDM, to obtain a cell suspension,
iv) mixing the resulting cell suspension with a methylcellulose medium [e.g. trade name: MethoCult GF+(manufactured by StemCell Technologies, Cat. No.: ST-04435) etc.], and stirring the mixture, and
v) injecting the resulting medium containing the cell into a dish, and culturing the cell for 14 days under the conditions of 37°C and 5% by volume CO₂.

In addition, expression of the marker gene specific for a primate of the (2) can be assessed by extracting a DNA by a conventional method from a colony formed in the hematopoietic colony assay, and for the resulting DNA, detecting a marker gene specific for a primate by hybridization using a probe specific for the gene, or PCR using a specific primer pair. Alternatively, the expression may be assessed by immunologically staining a hematopoietic colony with an antibody to an antigen specific for a primate.

Here, in the method of differentiation of the present invention, the cell obtained in the step (I) may be further transplanted into the sheep fetus obtained in the step (II), i.e., in more strict sense, a postnatal lamb. By further transplantation of the cell obtained in the step (I) into the postnatal lamb, a hematopoietic cell of a primate can be supplied efficiently and stably.

In addition, in the method of differentiation of the present invention, a cytokine or the like specific for a primate may be administered to the postnatal lamb obtained in the step (II). By administering the cytokine to the lamb, it becomes possible to selectively stimulate proliferation of a hematopoietic cell essentially derived from a primate in a body of the postnatal lamb.

The cytokine includes stem cell factor (SCF), basic fibroblast growth factor (bFGF), oncostatin M (OSM), flk2/flk3 ligand, interleukin-1, interleukin-3, granulocyte colony-stimulating factor (G-CSF), chemokine, erythropoietin, thrombopoietin and the like, and any combination thereof.

In the method of differentiation of the present invention, there is exhibited an excellent effect that a high chimera ratio is surprisingly accomplished by maintaining the cell under the conditions suitable for induction of differentiation into a hematopoietic cell for 6 days to 8 days, preferably for 6 days, transplanting the resulting cell into a fetus in a uterus of a pregnant sheep, rearing the fetus, and administering a cytokine specific for a primate to a born lamb.

Assessment of the hematopoietic cell of a primate obtained by the method of differentiation of the present invention can be performed, for example, using as an index, the presence or absence of expression of CD45, TER119, α₄-integrin, VE-cadherin, c-Kit, Sca-1, CD34 (stem cell marker), CD13 (myelocyte marker), CD14 (myelocyte marker), GpIIb/IIIa (megakaryocyte marker), CD3 (T cell marker), CD4 (T cell marker), CD8 (T cell marker), sIgM (B cell marker), CD19 (B cell marker) or the like.

The method of differentiation of the present invention can be used in production of a hematopoietic cell of a primate.

In other aspect, the present invention relates to a method for producing a hematopoietic cell of a primate.

The method for producing a hematopoietic cell of a primate of the present invention is a method comprising the steps of:
(I) maintaining an embryonic stem cell of a primate on a feeder cell, the feeder cell being a stromal cell strain deficient in macrophage colony-stimulating factor,
(II) transplanting the cell essentially derived from a primate obtained in the step

(I) into a fetus in a uterus of a pregnant sheep, and rearing the fetus to birth, and
(III) administering a cytokine specific for a primate to the fetus born in the step (II), i.e., in more strict sense, a lamb, and separating a hematopoietic cell of a primate differentiated from the embryonic stem cell of a primate from a sheep obtained by rearing the lamb.

One great feature of the method for production of the present invention is that the method comprises maintaining an embryonic stem cell of a primate under the conditions for inducing differentiation of a hematopoietic system. Therefore, according to the method for production of the present invention, since an embryonic stem cell of a primate is maintained under the aforementioned conditions for inducing differentiation of a hematopoietic system, surprisingly, there is exhibited an excellent effect that a hematopoietic cell can be produced from the embryonic stem cell of a primate, although differentiation from the undifferentiated embryonic stem cell into a hematopoietic cell is not substantially generated when the undifferentiated embryonic stem cell is transplanted as it is.

In addition, in the method of differentiation of the present invention, since when an embryonic stem cell of a primate is transplanted into a sheep fetus, the embryonic stem cell can more effectively utilize the internal environment of the sheep fetus (i.e. in the strict sense, after birth, a postnatal lamb), there is exhibited an excellent effect that a hematopoietic cell of a primate can be stably supplied.

In addition, one great feature of the method for production of the present invention resides also in use of a sheep fetus in a uterus of a pregnant sheep.

Since the sheep fetus is used, the method for production of the present invention exhibits an excellent effect that a chimera ratio with a primate is increased, thereby a hematopoietic cell of a primate can be supplied at a large scale from an embryonic stem cell of a primate.

In addition, according to the method for production of the present invention, in the step (I), by maintaining an embryonic stem cell, for example, a cynomolgus monkey embryonic stem cell strain CMK6 or the like under the conditions suitable for induction of differentiation into a hematopoietic cell for 6 days to 8 days, preferably for 6 days, it becomes possible to perform differentiation from an embryonic stem cell into a hematopoietic cell at a surprisingly high efficiency and more rapidity.

Further, according to the method for production of the present invention, since the same primate embryonic stem cell can be additionally transplanted into a postnatal lamb, and a cytokine of a primate which does not act on a sheep can selectively stimulate the transplanted primate embryonic stem cell or a hematopoietic cell essentially derived from a primate, a hematogenous cell essentially derived from a primate can be supplied more efficiently and stably.

In addition, according to the method for production of the present invention, platelet for transfusion and a CD34⁺cell for transplantation can be stably supplied.

The step (I) and the step (II) in the method for production of the present invention are similar to the step (I) and the step (II) in the method of differentiation of the present invention.

In the step (III), a hematopoietic cell of a primate differentiated from the embryonic stem cell of a primate is separated from a fetus born in the step (II), i.e., a lamb.

In the step (III), it is desirable that a sheep fetus, i.e., a lamb is an individual within one year after birth from the viewpoint of a chimera ratio.

In addition, it is preferable that, in the step (III), the cytokine specific for a primate or the like is administered to the born lamb and the lamb is further reared.

Further, like in the aforementioned differentiation method, from the viewpoint of supplying a hematopoietic cell of a primate efficiently and stably, the cell obtained in the step (I) may be further transplanted into the lamb born in the step (II), i.e., a postnatal lamb.

In the step (III), a hematopoietic cell of a primate can be separated, for example, by:
1) collecting liver, bone marrow, blood (peripheral blood, cord blood), thymus, spleen or the like from the sheep obtained as mentioned above;
2) obtaining a group of cells from the resulting organ by a proper procedure;
3) separating a hematopoietic cell derived from a primate from the resulting group of cells; and the like.

In the step 3), flow cytometry using a marker specific for a primate, an antibody to the marker or the like, a method using immunological beads, or the like is performed.

The "marker specific for a primate" may be a marker which crosses with a primate but does not cross with a sheep, and the marker includes HLA-ABC, β2-microglobulin, CD45 and the like.

Assessment of the hematopoietic cell of a primate obtained by the method for production of the present invention can be performed by using, as an index, for example, the presence or absence of expression of CD45, TER119, α₄-integrin, VE-cadherin, c-kit, Sca-1, CD34, CD13, CD14, GPIIb/IIIa, CD3, CD4, CD8, sIgM, CD19 or the like as in the case of the differentiation method.

In another aspect, the present invention relates to the hematopoietic cell of a primate obtained by the method for production of the present invention.

Such hematopoietic cell is expected to have utility, for example, in a transplantation material or the like for vascularization therapy without particular limitation, and application to an ischemic disease such as myocardial infarction becomes possible.

The hematopoietic cell of the present invention is specifically a hematopoietic stem cell and a group of cells generated by differentiation from the hematopoietic stem cell, i.e., cells having properties of erythroblast, myelocyte, megakaryocyte, lymphocyte or the like, specifically erythroblast, myelocyte or the like, as described above.

The hematopoietic cell of the present invention is a cell essentially derived from a primate, and it is a cell in which a foreign gene has not been introduced at production. Therefore, it is suitable for transplantation and the like to a primate.

By transplanting the hematopoietic cell of the present invention into a site of a disease or a condition requiring construction of a hematopoietic system or an action of a hematopoietic cell, there can be exhibited a therapeutic effect. Therefore, in another aspect, the present invention relates to a method of treating a disease requiring construction of a hematopoietic system or an action of a hematopoietic cell, characterized by supplying a therapeutically effective amount of the hematopoietic cell of the present invention to a site of a disease or a condition requiring construction of a hematopoietic system or an action of a hematopoietic cell. In still another aspect, the present invention relates to use of the hematopoietic cell of the present invention for treatment of a disease requiring construction of a hematopoietic system or an action of a hematopoietic cell.

The disease requiring construction of a hematopoietic system or an action of a hematopoietic cell is not particularly limited, but includes aplastic anemia, leukemia, immunodeficiency, ischemic heart disease such as myocardial infarction, arteriosclerosis obliterans, Paget disease, and the like.

The "therapeutically effective amount" may be a sufficient amount for constructing a hematopoietic system or showing an effect of a hematopoietic cell, and can be appropriately set depending on a degree of a disease or a condition requiring construction of a hematopoietic system or an action of a hematopoietic cell; weight, age, and physical strength of an individual; a degree of progression of construction of a hematopoietic system; and the like.

The hematopoietic cell of the present invention may be supplied to a site of a disease or a condition requiring construction of a hematopoietic system or an action of a hematopoietic cell, for example, by injection by puncture or the like, and if necessary, surgical operation or the like may be performed.

The therapeutic effect can be assessed by using as an index recognition of amelioration of symptom of the disease or amelioration of the condition by a suitable means (e.g. observation by fiberscope, ultrasound, CT or MRI, angiography, determination of a disease-specific marker in serum, or the like).

In addition, in another aspect, the present invention relates to use of the hematopoietic cell of the present invention for preparing a medicament for treating a disease requiring construction of a hematopoietic system or an action of a hematopoietic cell.

By a procedure similar to that of the differentiation method of the present invention, a chimeric sheep which produces a hematopoietic cell of a primate can be produced. Therefore, in still another aspect, the present invention relates to a method for producing a chimeric sheep which produces a hematopoietic cell of a primate.

One great feature of the method for producing a chimeric sheep which produces a hematopoietic cell of a primate of the present invention is that the method comprises maintaining an embryonic stem cell of a primate under the conditions suitable for induction of differentiation into a hematopoietic cell, transplanting the resulting cell into a fetus in a uterine of a pregnant sheep, administering a cytokine specific for a primate to a born lamb, and further rearing the lamb. There is exhibited an excellent effect that a chimeric sheep can be obtained at a surprisingly high efficiency by maintaining a cell under the conditions suitable for induction of differentiation into a hematopoietic cell for 6 days to 8 days, preferably for 6 days, transplanting the resulting cell into a fetus in a uterine of a pregnant sheep, rearing the fetus, and administering a cytokine specific for a primate to a born lamb.

According to the method for producing a chimeric sheep of the present invention, a chimeric sheep which can more stably supply a hematopoietic cell of a primate can be obtained.

Since the chimeric sheep obtained by the method for producing a chimeric sheep of the present invention is in a state of immunologic tolerance and has a cell essentially derived from a primate, a cell obtained by maintenance under the conditions suitable for induction of differentiation into a hematopoietic cell can be further transplanted into the chimeric sheep. In addition, in the chimeric sheep obtained by the method for producing a chimeric sheep of the present invention, by administering a cytokine specific for a primate, differentiation into a hematopoietic cell essentially derived from a primate can be selectively stimulated in a body.

The present invention will be explained in more detail hereinbelow by way of Examples, but the present invention is not limited to these Examples at all. In the following Examples, unless otherwise indicated, "%" indicates % by weight. In addition, the expression "%" in CO₂, O₂ and N₂ indicates % by volume.

### Example 1 Culturing of Monkey Embryonic Stem Cell

### (1) Preparation of Feeder Cell

Mouse embryo fibroblast BALB/cAJc1 (supplied by CLEA Japan, Inc.) subcultured up to five times was cultured in a D10 medium [composition: 10% FCS, DMEM] containing a final concentration 10 µg/ml of mitomycin C for 2 to 4 hours to stop mitotic proliferation of the cell and inactivate the proliferation of the cell. The medium containing mitomycin C was then removed. The cell after treatment was washed with a phosphate buffered saline. The cell after washing was treated with trypsin-EDTA (0.05% trypsin, 1 mM EDTA) to obtain a cell suspension, and the cell suspension was then centrifuged to obtain a cell.

Next, the resulting cell was suspended in a D10 medium, and plated on a gelatin-coated 6-cm culturing dish [manufactured by FALCON, trade name: Tissue culture dish] so that the number of the cells was to be 1×10⁶. The cell obtained by culturing until adhesion to the dish was used as a feeder cell.

### (2) Culturing of Cynomolgus Monkey-Derived ES Cell Strain CMK6

The D10 medium was removed from the culture of the feeder cell of item (1) of aforementioned Example 1, and the feeder cell was washed with a phosphate buffered saline.

Next, a cynomolgus monkey-derived ES cell CMK6 which had been stored with frozen was suspended in 5 ml of a medium for an ES cell [composition per 200 ml: 163 ml of DMEM-F12 (manufactured by GIBCO), 30 ml of fetal bovine serum (final concentration: 15%), 2 ml of L-glutamine (final concentration: 2 mM), 2 ml of penicillin (100 U/ml)-streptomycin (100 µg/ml), 2 ml of nonessential amino acid solution (manufactured by GIBCO), and 1 ml of 2-mercaptoethanol (final concentration: 0.1 mM)]. The resulting cell suspension was plated on the feeder cell. Here, culturing was carried out in an incubator under the conditions of 37°C and 5% CO₂. The medium for an ES cell was exchanged once every two days.

After culturing for 7 days, an old medium was removed by aspiration, and the cell was washed with a phosphate buffered saline. Next, 1 ml of 0.25% trypsin/HBSS (Hanks buffered saline solution) was added to the cell on the dish, and the cell was cultured at 37°C for 3 to 4 minutes. Thereafter, the bottom of the dish was lightly tapped at normal temperature to peel off a colony of the cell.

Next, a colony of an undifferentiated cell was recovered from the colony of the cell by pipetting with a 5-ml pipette using the medium for an ES cell. The recovered cell was suspended in the medium for an ES cell, and 5 ml of the resulting cell suspension was plated on a feeder cell on a 6-cm dish. Thereafter, the cell was subcultured every 2 to 4 days depending on the size of a colony.

The resulting undifferentiated ES cell was used in transplantation into a sheep fetus. In addition, such undifferentiated ES cell was used in the following induction of differentiation of a hematopoietic system.

### Example 2 Induction of Differentiation of a Hematopoietic System of Monkey Embryonic Stem Cell

### (1) Preparation of Feeder Cell

An OP9 cell which is a stromal cell strain prepared from newborn mouse calvaria of (C57BL/6×C3H) F2-op/op mouse having dysfunction in a macrophage colony-stimulating factor; and which is a preadipocyte strain, was cultured under the conditions of 37°C and 5% CO₂ in a medium for an OP9 cell [composition per 1250 ml: 980 ml of α-MEM (manufactured by Gibco, Cat. No.: 12000-022), 250 ml of fetal bovine serum (final concentration: 20%), 10 ml of penicillin (100 U/ml)-streptomycin (100 µg/ml), and 10 ml of 200 mM L-glutamine solution]. Thereafter, the cell which had been adhered in a culture medium, and which had been grown to confluent or immediately therebefore was washed twice with a phosphate buffered saline. Next, 0.1% trypsin-EDTA (2 ml/10-cm dish) was added to the washed cell on the dish, and the cell was incubated for 5 minutes in an incubator.

The medium for an OP9 cell was added to the dish, and a medium containing the cell was recovered in a 50-ml conical tube. The recovered medium containing the cell was centrifuged at 1500 rpm for 5 minutes. The resulting cell was plated on the medium for an OP9 cell at 1:4 to 1:5 (about 2.5×10⁵ cells to 4×10⁵ cells), and cultured under the conditions of 37°C and 5% CO₂ to confluent.

The resulting cell was cultured in 10 ml of the medium for an OP9 cell containing a final concentration 10 µg/ml of mitomycin C on a 10-cm dish (manufactured by FALCON) for 2 to 4 hours, to stop mitotic proliferation of the cell and inactivate the proliferation of the cell. The medium containing mitomycin C was then removed. The cell after treatment was washed with a phosphate buffered saline. The cell after washing was treated with trypsin-EDTA (0.05% trypsin, 1 mM EDTA) to obtain a cell suspension, and the cell suspension was centrifuged to obtain a cell.

The resulting cell was plated on the medium for an OP9 cell at 1:2 (about 1×10⁵ cells/ml per medium), on a gelatin-coated 6-cm culturing dish (manufactured by FALCON, trade name: Tissue Culture Dish). The cell thus prepared was used as a feeder cell in the following culturing for inducing differentiation of a hematopoietic system.

### (2) Culturing for Inducing Differentiation of a Hematopoietic System

The cynomolgus monkey-derived ES cell strain CMK6 strain recovered in 1 ml of 0.25% trypsin/HBSS in aforementioned Example 1 was suspended in 5 ml of a medium for differentiation [composition per 100 ml: 84 ml of IMDM, 8 ml of 8% horse serum, 8 ml of 8% fetal bovine serum, 0.18 µg of 5×10⁻⁶ M hydrocortisone, 2 µg of BMP-4 (final concentration: 20 ng/ml), 2 µg of SCF (final concentration: 20 ng/ml), 2 µg of IL-3 (final concentration: 20 ng/ml), 1 µg of IL-6 (final concentration: 10 ng/ml), 2 µg of VEGF (final concentration: 20 ng/ml), 2 µg of G-CSF (final concentration: 20 ng/ml), 2 µg of Flt.3 ligand (final concentration: 10 ng/ml), and 200 U of EPO (final concentration: 2 U/ml)], and the resulting cell suspension was plated on the feeder cell in the culturing for inducing differentiation of a hematopoietic system obtained in item (1) of aforementioned Example 2 at 1:3.

Thereafter, the cell was cultured for 6 days under the conditions of 37°C and 5% CO₂. Here, during culturing, the medium was appropriately exchanged. The resulting cell (Fig.1) was used in the following transplantation and the like as a treated cell for inducing differentiation of a hematopoietic system.

### (3) Preparation of Cell for Transplantation

The medium was aspirated from the dish of the treated cell for inducing differentiation of a hematopoietic system obtained in item (2) of aforementioned Example 2, 1 ml of 0.25% trypsin/HBSS was added to the dish, and the cell was cultured for 4 minutes under the conditions of 37°C and 5% CO₂.

Next, the cell on a feeder cell together with the feeder cell was recovered in a 50 ml-volume conical tube containing 20 ml of the medium for differentiation with a scraper (trade name: cell scraper-M, manufactured by SUMILON), and the mixture was centrifuged at 800 rpm for 4 minutes.

The supernatant was aspirated, and the cell was suspended in a 50 ml-volume conical tube containing 20 ml of the medium for differentiation to obtain a cell suspension.

Immediately before transplantation, the cell suspension was centrifuged at 800 rpm for 4 minutes. The supernatant was aspirated, and 20 ml of 0.1% BSA/HBSS was then added to the resulting cell to suspend the cell, and the suspension was washed by centrifugation at 800 rpm for 4 minutes.

Thereafter, the supernatant was aspirated, and the cell (1×10⁷ to 1 ×10⁸ cells) was suspended in 0.4 ml of 0.1% BSA/HBSS. The resulting suspension was placed in a 1.5-ml assist tube, and the suspension was stored on ice until transplantation.

### Example 3 Transplantation of Cell in Sheep Uterine

A pregnant sheep purchased from an experimental sheep dealer Japan Lamb was used. Transplantation was scheduled on about 60th day of pregnancy, and the sheep was acclimated to the breeding environment at transplantation, one week to 10 days before the transplantation. In addition, the presence of a fetus was confirmed by an abdominal ultrasonography.

The maternal sheep was anesthetized by intramuscular injection of ketamine (15 mg/kg weight). The sheep was laid on its back on an operating table, four limbs were fixed, and orotracheal intubation was carried out, and an operation was performed under general anesthesia with O₂/air/halothane and under spontaneous respiration.

The operation was performed by a clean procedure. After celiotomy, by hypogastric middle incision, uterine was turned into an abdominal cavity.

There were set two kinds of sites for injection of the cell for transplantation, I) in abdominal cavity and II) in hepatic parenchyma of a fetus.

There were set two ways of methods for transplantation of the cell, I) hysterotomy and II) ultrasonic guide method. In the case of hysterotomy, a myometrium of a sheep was incised, a sheep fetus was driven into a velamen which was exposed with preserved, the cell for transplantation (1×10⁶ to 1×10⁸ cells) was injected by puncture into a fetal abdominal cavity through a transparent velamen with a 23G needle under opening, and the myometrium, a peritoneal muscle layer and a skin were sequentially sutured to close an abdomen. In addition, in the case of ultrasonic guide method, without incising a uterine, the cell for transplantation (1×10⁷ to 1×10⁸ cells) was injected by puncture into a sheep fetal hepatic parenchyma with a 25G catelan needle from above a uterine wall under ultrasonic guide, and a peritoneal muscle layer and a skin were sequentially sutured to close an abdomen.

After the operation, an antibiotic (mycillin sol) was intramuscularly injected. Extubation was then carried out, and revival from the anesthetic of a sheep was confirmed.

Persistency ratio of pregnancy after hysterotomy and ultrasonic guide method was 40% (4/10) and 80% (8/10), respectively, and a high persistency ratio of pregnancy was obtained in an ultrasonic guide method.

### Example 4 Collection of Sample from Sheep Fetus

A fetus one month after transplantation was taken out by cesarean section under general anesthesia of the maternal sheep and dissected, and a systemic tissue including a hematopoietic tissue was collected as a sample.

Specifically, from the maternal sheep with a total of four fetuses, two fetuses [transplanted fetus (tail cut), control fetus] in each of left and right uterine, collection and treatment of a tissue were performed simultaneously by the following procedure.

Anesthesia introduction was initiated to a maternal sheep by oral intubation under spontaneous respiration and O₂/air/halothane. After 15 minutes, the maternal sheep was subjected to cesarean section, two fetuses in a unilateral uterine were taken out, and a tissue was collected and treated. The weight of the fetus into which a cell was transplanted (transplanted fetus) was 950 g, and the weight of the control fetus was 1040 g.

With umbilical cord connected to the maternal sheep, umbilical venous blood (cord blood sample) and umbilical artery blood (peripheral blood sample) were collected. Here, the amount of the collected blood was 10 ml for the cord blood sample of the transplanted fetus, 20 ml for the peripheral blood sample of the transplanted fetus, 20 ml for the cord blood sample of the control fetus, and 20 ml for the peripheral blood sample of the control fetus.

An umbilical cord was separated, and an umbilical vein was then cannulated with a 6G atom tube. In an umbilical artery, since cannulation could not be performed, a separated end was opened.

A refluxing solution (500 ml of cold Lactec) was connected to a cannulae on the umbilical vein side, and refluxing was initiated by instillation. The umbilical artery side was used as a path to let the blood out, and refluxing was performed until blood was not flown out (around 1000 ml in total).

Each of the transplanted fetus and the control fetus was subjected to celiotomy, and partial liver, and thigh bone were collected as a sample for primary culture by a clean procedure. In addition, an organ was isolated. The isolated organ was separated and placed in a Petri dish on ice.

The collected tissues are shown below:

### Endodermal tissue

1. Liver (including bile duct, hepatic parenchyma, hepatic artery, and portal region)
2. Head of pancreas (pancreatic duct, pancreatic parenchyma)
3. Thymus
4. Thyroid gland
5. Lung (bronchus, alveolus)
6. Small intestine
7. Greater omentum (visceral peritoneum)

### Ectodermal tissue

8. Cerebrum
9. Spinal cord
10. Skin (appendage; hair, sweat gland, sebaceous gland)
11. Tail-part cut wound (skin)

### Mesodermal tissue

12. Fat (connective tissue)
13. Cartilage (epiphysial cartilage)
14. Bone marrow
15. Lymph node
16. Skeletal muscle
17. Cardiac muscle
18. Aorta
19. Spleen
20. Kidney
21. Gonad

For primary culture, the following samples were collected:
22. Peripheral blood (umbilical arterial blood)
23. Cord blood (umbilical venous blood)
24. Bone marrow (after refluxing)
25. Liver (after refluxing)

Three samples of a tissue specimen of 5 mmx 5 mmx 3 mm were excised from each of the collected tissues. Among them, two samples were placed into a 15-ml sample bottle containing 4% buffered paraformaldehyde (PFA)/8% sucrose, and fixed overnight. Such sample was defined as a 4% PFA-fixed sample.

In addition, a bone marrow sample was subjected to decalcification.

In addition, one sample of the 4% PFA-fixed sample was sequentially maintained in 50% ethanol, 60% ethanol, 70% ethanol, 80% ethanol, and 90% ethanol for 1 to 2 hours, maintained in 100% ethanol for 1 to 2 hours, further maintained in 100% ethanol for 1 to 2 hours, further maintained in 100% ethanol overnight to dehydrate, immersed in benzene (30 minx2), and then immersed in paraffin (45 minx2), thereby a sample of paraffin-embedded section after 4% PFA-fixation was obtained.

Another sample of the 4% PFA-fixed samples was placed in a cryomold 1 (Tissuetek, Miles, Elkhart, IN, USA) and embedment-frozen. The embedment freezing was performed by embedding the sample in an OCT compound (Tissuetek), and freezing with liquid nitrogen. Such sample was used as a specimen for a section frozen after fixation.

The remaining one sample was also placed in the same cryomold 1 and embedment-frozen. The embedment freezing was performed by embedding the sample in an OCT compound, and freezing with liquid nitrogen. Such sample was used as a specimen for a freshly frozen section.

Some small pieces were excised from the remaining collected tissues, each one of them was placed in three cryotubes, and each tube was frozen with liquid nitrogen. A small piece of these small organs was used as a specimen for DNA extraction or RNA extraction.

In addition, in the case of abortion, stillbirth or postnatal death, a fetus was taken out, and about 20 ml of peripheral blood (heparin blood collection), a suitable amount of bone marrow [collected in heparin physiological saline (10 U/ml)], about 20 ml of cord blood (heparin blood collection), and about 10 g of liver (collected in heparin physiological saline) were collected. A body surface, the inside of an abdominal cavity, a thoracic cavity and a brain were subjected to necropsy to confirm whether a tumor such as teratoma was formed or not, but formation of a tumor was not recognized. Here, in a birth example, formation of a tumor is not recognized.

In addition, in the case where analysis was performed after birth, peripheral blood and bone marrow were collected at a frequency of about once per month after birth. Specifically, after birth, about 20 ml of peripheral blood (heparin blood collection) was collected every two weeks in a first few months, and a suitable amount of bone marrow (collected in heparin physiological saline) was collected every one month. Collected samples were placed into a sterile conical tube and retained while shaking at room temperature, and a nucleated cell was separated within one day, and frozen and stored.

Regarding a hematopoietic tissue and cell such as liver, cord blood, bone marrow and peripheral blood, a leukocyte fraction was separated by a hemolysis method, and frozen and stored at -80°C or in liquid nitrogen, as a sample for DNA extraction or RNA extraction, a sample for primary culture, or a sample for FACS analysis.

In addition, regarding liver, as a sample for primary culture, a cell was isolated as follows. A sample was collected under a sterile procedure, the sample was suitably cut into strips, and recovered in a 50-ml conical tube. Twenty milliliters of 1% trypsin/EDTA/phosphate buffered saline was added thereto and suspended well, and culturing was performed at 37°C for 10 minutes while shaking. Two-hundred-microliters of a DNase I solution [solution in which 10 mg of DNase I was dissolved in 1 ml 0.15 M Nacl] was added to the resulting product, and 100 µl of 1 M MgCl₂ was further added thereto, followed by culturing at 37°C for 10 minutes. Thereafter, the product was filtered with a nylon mesh having a diameter of 70 mm (manufactured by FALCON, trade name: cell strainer), and a D10 medium was added in the same amount as that of the product. Thereafter, the mixture was centrifuged at 1500 rpm for 5 minutes, and the supernatant was aspirated. Here, when a pellet was red due to contamination of erythrocyte, an about 10-fold amount or more of an ACK-hemolysis medium was added to the pellet, and the mixture was maintained on ice for 15 to 0 minutes, and centrifuged at 1500 rpm at 4°C for 5 minutes. The pellet was washed twice with about 40 ml of a phosphate buffered physiological saline. The supernatant was aspirated, and the resulting pellet was suspended in a cell banker at 10⁶ to 10⁷ cells/vial, and frozen and stored at -80°C or in liquid nitrogen.

In addition, regarding cord blood, peripheral blood, and bone marrow, as a sample for primary culture, a cell was isolated as follows: a sample was collected by a sterile procedure by the same method as in the heparin blood collection, 35 ml of blood was placed into a 50-ml conical tube, and the blood was centrifuged at 1500 rpm for 10 minutes. Plasma was aspirated, and a pellet was dispensed into five 50-ml conical tubes. Then, 40 ml (about 10-fold amount of pellet) of an ACK-hemolysis medium was added to each conical tube, and these were kneaded, followed by culturing on ice for 15 minutes. After centrifugation at 1500 rpm at 4°C for 5 minutes, the supernatant was aspirated. In addition, when the pellet was red, centrifugation at 1500 rpm for 10 minutes was repeated. Then, 40 ml of a phosphate buffered physiological saline was added to the pellet to suspend it, five tubes were unified into one tube, and the pellet was washed by centrifugation at 1500 rpm at 4°C for 5 minutes. Such washing was performed twice. The supernatant was aspirated, the pellet was suspended in a cell banker at 5×10⁶ cells/vial/ml, and the suspension was frozen and stored at -80°C or in liquid nitrogen.

### Example 5 Analysis of Monkey/Sheep Chimera of Each Tissue

Using trade name: QIAamp DNA Mini Kit or trade name: QIAamp DNA Blood Mini Kit, a DNA was extracted from the sample obtained in aforementioned Example 4 according to a protocol of a manufacturer attached to the kit.

For analyzing a sheep fetus into which a cell derived from a monkey had been transplanted, using as primer pairs for monkey β2-microglobulin, a primer pair consisting of an external primer pair:
CB2MG-2F: 5'-GTCTGGATTTCATCCATCTG-3' (SEQ ID NO: 1) and
hB2MG-5R: 5'-GGCTGTGACAAAGTCACATGG-3' (SEQ ID NO: 2), and
a primer pair consisting of an internal primer pair:
CB2MG-2F and
hB2MG-3R: 5'-GGTGAATTCAGTGTAGTACAAG-3' (SEQ ID NO: 3), and using the DNA as a template, nested PCR (two-stage PCR method in which after PCR using an external primer pair, PCR using an internal primer pair is performed) was performed. In addition, PCR conditions were as follows: Using a reaction solution [composition: 30.75 µl of H₂O, 5 µl of 10×PCR buffer, 4 µl of dNTP (2.5 mM each), 0.25 µl of Ex Taq (5 U/ µl), 2.5 µl each of primers (10 pM each), and 5 µl of sample-derived DNA (amount corresponding to 250 ng)], after a reaction at 95°C for 5 minutes, 25 cycles were performed at amplification using an external primer pair, and 30 cycles were performed at amplification using an internal primer pair, each cycle consisting of one minute at 95°C, one minute at 58°C and one minute at 72°C. Thereafter, a reaction was performed at 72°C for 5 minutes, and a temperature was maintained at 4°C.

In addition, using a primer pair consisting of a primer pair for β-actin:
Cβ1: 5'-CATTGTCATGGACTCTGGCGACGG-3' (SEQ ID NO: 4) and
Cβ2: 5'-CATCTCCTGCTCGAAGTCTAGGGC-3' (SEQ ID NO: 5),
PCR (30 cycles, one cycle consisting of 1 minute at 95°C, 1 minute at 54°C and
2 minutes at 72°C), were performed with a similar reaction solution.

Here, for quantification, samples of dilution series shown in Table 1 were prepared, and used.

**Table 1**

| No. | 9 | 8 | 7 | 6 | 5 | 4 | 3 | 2 | 1 |
|---|---|---|---|---|---|---|---|---|---|
| Dilution Ratio (%) | 100 | 10 | 1 | 0.1 | 0.01 | 0.001 | 0.0001 | 0 | H₂O |
| Monkey DNA(µl) | 100 | 10 | 10 | 10 | 10 | 10 | 10 | - | - |
| Sheep DNA(µl) | - | 90 | 90 | 90 | 90 | 90 | 90 | 90 | - |
| Total amount (µl) | 90 | 90 | 90 | 90 | 90 | 90 | 90 | 90 | - |

The product produced by PCR was subjected to electrophoresis with 2% agarose gel (containing 0.01% ethidium bromide).

Here, in each tissue of a sheep fetus one month after transplantation, a cell derived from a monkey could not be detected, and as described in the following Example 6, a cell derived from a monkey was detected only in a hematopoietic precursor cell of a sheep into which a cell obtained by induction of differentiation of a hematopoietic system for a monkey embryonic stem cell had been transplanted. Therefore, the present method is particularly suitable for differentiation from a primate embryonic stem cell into a hematopoietic cell, and it is thought that cell differentiation of other lineage does not occur. Thus, it is suggested that the present method is a method excellent in specificity for hematopoietic differentiation.

### Example 6 Analysis of Monkey/Sheep Hematopoietic Chimera

A hematopoietic colony assay was performed for a cell which had been separated for primary culture.

A cryotube of the frozen and stored cell was thawed in a thermostatic chamber at 37°C, and suspended in a 15-ml conical tube containing 9 ml of 2% FBS-IMDM, and a part of the resulting suspension was used to count the number of cells. Then, the suspension was centrifuged at 1300 rpm for 4 minutes, and the resulting pellet was suspended in 2% FBS-IMDM so as to have a density of 1 × 10⁶ cells/ml, to obtain a cell suspension. Further, the suspension was diluted 10-fold with 2% FBS-IMDM to prepare a 1×10⁵ cells/ml cell suspension.

Four milliliters of a methylcellulose medium (trade name: Methocult GF+ [manufactured by StemCell Technologies, Cat. No.: ST-04435]) was placed into a 14-ml polystyrene round-bottom tube, 400 µl of the cell suspension was added thereto, a cap was closed, and the mixture was well-shaken to stir. Thereafter, the mixture was allowed to stand for about 5 minutes until foams disappeared.

Next, 1.1 ml of the resulting medium containing a cell was injected into a centre of a 3.5-mm dish with a 2.5-ml injector equipped with a 18G needle, and the medium was uniformly spread on the bottom of the dish.

Culturing was performed under the conditions of 37°C and 5% CO₂. A colony was counted on 14th day from initiation of culturing.

In such method, since a hematopoietic precursor cell derived from a monkey and a hematopoietic precursor cell derived from a sheep have an equivalent colony forming ability, a ratio of the number of colonies derived from a monkey to the total number of colonies indicates a hematopoietic chimera ratio.

For identifying a colony derived from a monkey, colonies were taken from the medium on which colonies were formed, a DNA was extracted from each colony, PCR was performed by employing the resulting DNA as a template and using primer pairs for monkey β2-microglobulin, and a monkey-derived colony was confirmed.

Using an external primer pair: a primer pair consisting of the aforementioned CB2MG-2F and hB2MG-5R, and an internal primer pair: a primer pair consisting of the aforementioned CB2MG-2F and hB2MG-3R as primer pairs for monkey β2-microglobulin, and employing the DNA as a template, nested PCR was performed. Here, PCR conditions were as follows: Using a reaction solution [composition: 30.75 µl of H₂O, 5 µl of 10xPCR buffer, 4 µl of dNTP (2.5 mM each), 0.25 µl of trade name: Ex Taq (5 U/µl), 2.5 µl each of primers (10 pM each), and 5 µl of sample-derived DNA (an amount corresponding to 250 ng)], after a reaction at 95°C for 5 minutes, 25 cycles were performed in both at amplification using an external primer pair and at amplification using an internal primer, each cycle consisting of 30 seconds at 94°C, 1 minute at 57°C and 1 minute at 72°C. Thereafter, a reaction at 72°C for 5 minutes, and the temperature was maintained at 4°C. The resulting product was analyzed by electrophoresis with a 2% agarose gel.

As a result, when a monkey ES cell of which differentiation into a hematopoietic system is induced is used, as shown in Fig.2, a hematopoietic colony derived from a monkey was detected at a frequency of about 30% (n=1) in a fetus liver of an individual one month after transplantation of an ES cell treated with induction of differentiation of a hematopoietic system into an abdominal cavity, and at a frequency of about 1% (n=3) in bone marrow of an individual after birth (3 to 6 months after transplantation) into which an ES cell treated with induction of differentiation of a hematopoietic system had been transplanted to hepatic parenchyma. In other words, by ES cell of which differentiation into a hematopoietic system is induced and introducing the cell into a sheep fetus, a born sheep produces a hematopoietic cell derived from a monkey. On the other hand, in a sheep into which an undifferentiated monkey embryonic stem cell had been transplanted, a hematopoietic colony forming cell derived from a monkey could not be detected in liver one month after transplantation (n=2). Therefore, it was seen that the culturing for inducing differentiation of a hematopoietic system of an embryonic stem cell described in Example 2 is essential in producing a monkey/sheep hematopoietic chimera.

### Example 7 Study of the Effect of Cytokine on Hematopoietic Chimera Ratio

One-thousand micrograms of recombinant human stem cell factor (rhSCF) [1.5 mg/ml, manufactured by Amgen Ltd., lot No. 15701F4] was diluted with 5 ml of a diluting solvent [composition: 10% sheep autologous serum and 0.1 U heparin sodium in HBSS], the resulting dilution was filtered with a 0.22-µm filter, and the filtered dilution was injected into an individual (n=2) in which a monkey/sheep chimera had been recognized in bone marrow colony PCR after birth, intraperitoneally using an injector equipped with a 23G needle at 60 µg/kg/day once a day for 18 days.

Sheep No: 141, which was a subject for administration, was 81 days old (156 days after transplantation), and the weight was about 14 kg at initiation of administration. In addition, sheep No: 182, which was a subject for administration, was 12 days old (94 days after transplantation), and the weight was about 10 kg at initiation of administration.

In each of 6 day after administration, 1 day after final administration and 3 day after final administration, peripheral blood and bone marrow were collected.

Three milliliters of a FACS buffer [composition: phosphate buffered physiological saline containing 5% fetal bovine serum and 0.05% NaN₃] was added to each cell to suspend the cell, and the resulting suspension was passed through a 70-µm mesh. The resulting product was centrifuged at 1500 rpm (490 xg) for 4 minutes, and the supernatant was aspirated. Thereafter, 450 µl of the FACS buffer was added to the resulting pellet.

Each 150 µl of the resulting solution was dispensed into a 96-well plate. Here, a sample obtained by adding 350 µl of the FACS buffer to 150 µl of the aforementioned solution was used as a negative control for antibody.

The solution in a well of a 96-well plate was then centrifuged at 1800 rpm (710 xg) for 2 minutes, and the supernatant was removed.

Thereafter, 20 µl of trade name: PE-Anti-human CD45 (manufactured by Becton, Dickinson and Company, BD Cat. No.: 557059) and 80 µl of the FACS buffer were added to a well. Here, in place of the PE-Anti-human CD45, as an isotype control, trade name: PE-Anti-mouse IgG1κ (manufactured by Becton, Dickinson and Company, Cat. No.: 33815X) was used. The resulting mixture was incubated on ice for 30 minutes, and thereafter centrifuged at 1800 rpm (710 xg) for 2 minutes, and the supernatant was removed. Thereafter, the resulting pellet was washed twice with 150 µl of the FACS buffer.

The resulting pellet was suspended in 350 µl of the FACS buffer, and the resulting sample was subjected to flow cytometry.

As a result, a CD45⁺ cell was not found.

Next, a leukocyte fraction was separated from peripheral blood and bone marrow by a hemolysis method in the same manner as described in Example 4. Using trade name: QIAamp DNA Mini Kit and trade name: QIAamp DNA Blood Mini Kit, a DNA was extracted from the resulting sample according to a protocol of a manufacturer attached to the kit. Next, employing the DNA as a template, and using an external primer pair of the CB2MG-2F and hB2MG-5R, and an internal primer pair of the CB2MG-2F and hB2MG-3R as primer pairs for monkey β2-microglobulin, nested PCR was performed. Here, PCR conditions were as follows: Using a reaction solution [composition: 30.75 µl of H₂O, 5 µl of 10xPCR buffer, 4 µl of dNTP (2.5 mM each), 0.25 µl of trade name: Ex Taq (5 U/ µl), 2.5 µl each of primers (10 pM each), and 5 µl of sample-derived DNA (an amount corresponding to 250 ng), after a reaction at 95°C for 5 minutes, 25 cycles were performed at amplification using an external primer pair, and 25 cycles were performed at amplification using an internal primer pair, each cycle consisting of 1 minute at 95°C, 1 minute at 58°C and 1 minute at 72°C. Thereafter, a reaction was performed at 72°C for 5 minutes, and the temperature was maintained at 4°C. The product produced by PCR was subjected to electrophoresis with a 2% agarose gel (containing 0.01% ethidium bromide), and observed. The results are shown in the upper panel of Fig.3. Next, a DNA on the gel was transferred to a PVDF membrane (manufactured by Amersham Pharmacia). Southern blot hybridization was performed on the resulting membrane using a probe for monkey β2 microglobulin prepared by PCR using primers having a nucleotide sequence shown in SEQ ID NO: 3 or a nucleotide sequence shown in SEQ ID NO: 4. The results are shown in the lower panel of Fig.3.

Here, as a control, for an amount corresponding to 250 ng of each of the following DNA:
only a sheep DNA (in Fig.3, lane 4),
a mixture of 0.0001% monkey DNA and 99.9999% sheep DNA (in Fig.3, lane 5),
a mixture of 0.001% monkey DNA and 99.999% sheep DNA (in Fig.3, lane 6),
a mixture of 0.01% monkey DNA and 99.99% sheep DNA (in Fig.3, lane 7),
a mixture of 0.1% monkey DNA and 99.9% sheep DNA (in Fig.3, lane 8),
a mixture of 1% monkey DNA and 99.9% sheep DNA (in Fig.3, lane 9),
a mixture of 10% monkey DNA and 90% sheep DNA (in Fig.3, lane 10), and
a monkey DNA alone (in Fig.3, lane 11),
PCR was performed simultaneously under the same conditions as for the specimen sample, and the product was used in electrophoresis.

As a result, from Fig.3, it was shown that a monkey cell appeared at a ratio of about 0.01% in peripheral blood on day 6 after SCF administration, and in bone marrow on day 1, day 3 and day 40 after administration.

Further, a sample for primary culture was prepared from each of peripheral blood and bone marrow by the same method as that of aforementioned Example 4. The resulting sample for primary culture was cultured on a methylcellulose medium [MethoCult GF+ (manufactured by Stem Cell Technologies)] for 14 days.

The resulting colony was collected, and a DNA was extracted from each colony. Employing the resulting DNA as a template and using the same primer pairs as that for monkey β2-microglobulin as that described above, PCR was performed. The results are shown in Table 2.

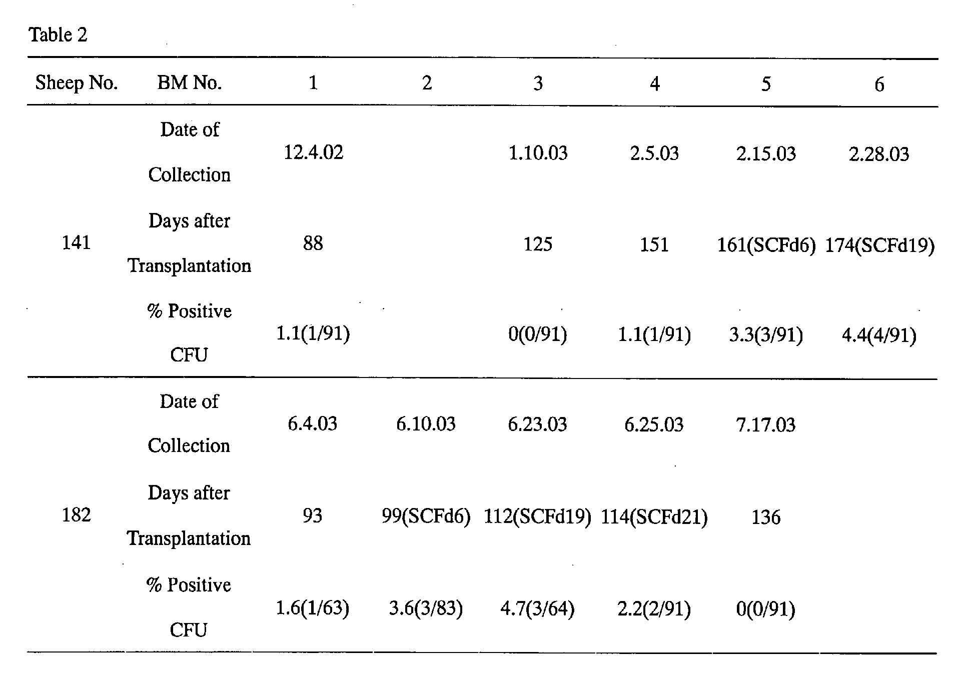

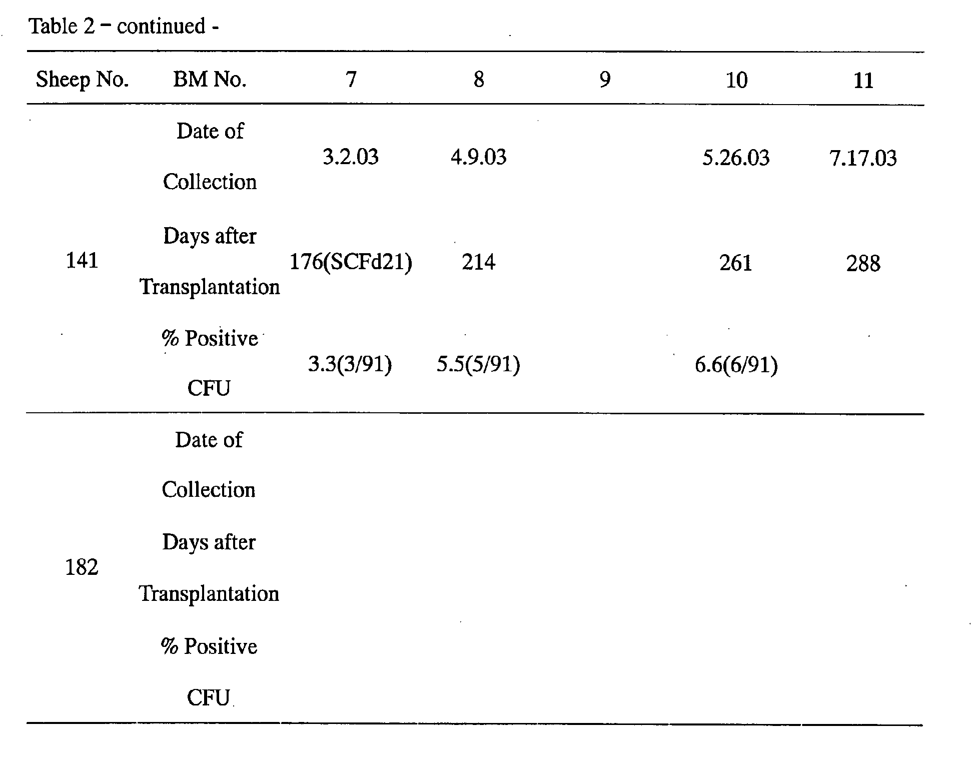

As a result, as shown in Table 2, it was found that a chimera ratio was increased at least 3- to 4-fold as compared to that before administration of a human stem cell factor.

### SEQUENCE LISTING FREE TEXT

SEQ ID NO: 1 is a sequence of primer CB2MG-2F.
SEQ ID NO: 2 is a sequence of primer hB2MG-5R.
SEQ ID NO: 3 is a sequence of a primer hB2MG-3R.
SEQ ID NO: 4 is a sequence of primer C beta 1.
SEQ ID NO: 5 is a sequence of a primer C beta 2.

### INDUSTRIAL APPLICABILITY

According to the present invention, it becomes possible to supply a cell which can be used for treating a disease or a condition requiring construction of a hematopoietic system or an action of a hematopoietic cell at a large amount, and development of a means for treating a disease or a condition requiring construction of a hematopoietic system or an action of a hematopoietic cell is expected.

## Claims

1. A method of differentiation from an embryonic stem cell of a primate into a hematopoietic cell, **characterized by** maintaining an embryonic stem cell of a primate under conditions suitable for induction of differentiation into a hematopoietic cell, transplanting the resulting cell into a fetus in a uterus of a pregnant sheep, rearing the fetus, administering a cytokine specific for a primate to a born lamb, and obtaining a hematopoietic cell of a primate from a sheep obtained by rearing the lamb.

2. The method according to claim 1, wherein the method comprises the steps of:
(I) maintaining an embryonic stem cell of a primate on a feeder cell, the feeder cell being a stromal cell strain deficient in macrophage colony-stimulating factor, and
(II) transplanting a primate-derived cell obtained in the step (I) into a fetus in a uterus of a pregnant sheep, and rearing the fetus to birth.

3. The method according to claim 2, wherein in the step (I), an embryonic stem cell of a primate is maintained on a feeder cell in the presence of bone morphogenetic protein 4.

4. A method for producing a hematopoietic cell of a primate, comprising the steps of:
(I) maintaining an embryonic stem cell of a primate on a feeder cell, the feeder cell being a stromal cell strain deficient in macrophage colony-stimulating factor,
(II) transplanting a primate-derived cell obtained in the step (I) into a fetus in a uterus of a pregnant sheep, and rearing the fetus to birth, and
(III) administering a cytokine specific for a primate to a lamb born in the step (II), and separating a hematopoietic cell of a primate differentiated from the embryonic stem cell of a primate from a sheep obtained by rearing the lamb.

5. A hematopoietic cell obtained by the method as defined in claim 4.

6. A method for producing a chimeric sheep which produces a hematopoietic cell of a primate, **characterized by** maintaining an embryonic stem cell of a primate under conditions suitable for induction of differentiation into a hematopoietic cell, transplanting the resulting cell into a fetus in a uterus of a pregnant sheep, administering a cytokine specific for a primate to a born lamb, and rearing the lamb.
